# EUROPEAN PATENT SPECIFICATION

(11) **EP 2 917 719 B1**
(45) Date of publication and mention of the grant of the patent: **17.04.2024**
(21) Application number: 12790422.5
(22) Date of filing: 09.11.2012
(51) Int. Cl.: G01N 21/03

(54) **RECEPTACLE AND SYSTEM FOR OPTICALLY ANALYZING A SAMPLE WITHOUT OPTICAL LENSES**
BEHÄLTER UND SYSTEM ZUR OPTISCHEN ANALYSE EINER PROBE OHNE OPTISCHE LINSEN
RÉCEPTACLE ET SYSTÈME POUR L'ANALYSE OPTIQUE D'UN ÉCHANTILLON SANS LENTILLES OPTIQUES

(43) Date of publication of application: 16.09.2015
(73) Proprietor: Fraunhofer-Gesellschaft zur Förderung der angewandten Forschung e.V., 80686 München (DE)
(72) Inventor: RAPOPORT, Daniel, Hans, 23564 Lübeck (DE); BECKER, Tim, 23611 Bad Schwartau (DE); KRUSE, Charli, 23923 Herrnburg (DE)
(74) Representative: v. Bezold & Partner Patentanwälte - PartG mbB
(86) International application number: PCT/EP2012/004668
(87) International publication number: WO 2014/071962

(56) References cited:
- EP-A1- 1 859 866
- WO-A1-2010/106989
- US-A1- 2002 155 617
- US-A1- 2012 046 187
- US-A1- 2012 223 217
- US-A1- 2012 224 053
- US-B1- 6 362 887
- Guoan Zheng ET AL: "The ePetri dish, an on-chip cell imaging platform based on subpixel perspective sweeping microscopy (SPSM)", Proceedings of the National Academy of Sciences of the United States of America, 11 October 2011 (2011-10-11), pages 16889-16894, XP055185863, United States DOI: 10.1073/pnas.1110681108 Retrieved from the Internet: URL:http://www.jstor.org/stable/41321803
- Seung Ah Lee ET AL: "On-chip continuous monitoring of motile microorganisms on an ePetri platform", Lab on a Chip, vol. 12, no. 13, 1 January 2012 (2012-01-01), page 2385, XP055396649, ISSN: 1473-0197, DOI: 10.1039/c2lc40090a

## Description

### Field of the invention

The invention relates to a receptacle for receiving a sample, e.g. a biological sample like a cell culture, during an optical analysis of the sample. Further, the invention relates to a system for optically analyzing a sample, e.g. a biological sample like a cell culture.

### Background of the invention

The state of the art comprises the so-called time-lapse microscopy which is used for life-cell imaging. The conventional time-lapse microscopy systems comprise an optical microscope, a digital camera, a computer software and an incubator to control the cellular environment of the sample. However, the conventional time-lapse microscopy systems are very expensive and complex. Further, it is difficult to integrate time-lapse microscopy systems into working environments in laboratories since they are too big and too complex for an integration into a conventional incubator. Finally, it is difficult to use the conventional time-lapse microscopy systems in connection with automated cell cultures ("cell farms"), which are needed in clinical applications.

Further, the state of the art comprises so-called Petri dishes which are receptacles for receiving a sample during an optical analysis of the sample, for example during the afore-mentioned time-lapse microscopy.

Moreover, a so-called ePetri dish is disclosed in Zheng et al.: "The ePetri dish, an on-chip cell imaging platform based on sub-pixel perspective sweeping microscopy (SPSM)", Proceedings of the national Academy of Sciences of the United States of America (PNAS) 2011. According to this idea, cell cultures are directly placed on the surface of a CMOS image sensor without any optical lenses in between. However, the CMOS image sensor is contaminated by the direct contact with the cell culture. Therefore, each measurement of a cell culture needs a new CMOS image sensor or a thorough cleaning of a used CMOS image sensor.

With regard to the general technical background of the invention, reference is also made to US 2002/155617 A1, EP 1 859 866 A1, US 2012/224053 A1 and WO 2010/106989 A1.

### Summary of the invention

Therefore, it is an object of the invention to provide an improved receptacle for optically analyzing a sample, e.g. a biological sample like a cell culture.

This object is achieved by a receptacle according to the independent claim.

Firstly, the invention provides a novel receptacle for receiving a sample during an optical analysis of the sample, wherein the receptacle comprises a bottom which is at least partially transparent, so that the sample within the receptacle can be optically analyzed by an image sensor from below the bottom.

In contrast to the conventional Petri dishes, the receptacle according to the invention comprises a very thin bottom with a thickness of less than 500 pm, 200 pm, 150 µm or even less than 120 µm. The thin bottom of the receptacle advantageously allows the use of the so-called "shadow imaging" for optically analyzing the sample within the receptacle. During the so-called shadow imaging, the receptacle with the sample is placed directly on the photosensitive area of an image sensor (e.g. a CCD sensor or a CMOS sensor) without any optical lens between the receptacle and the image sensor. It is important to have a very low thickness of the bottom of the receptacle in order to improve contrast and sharpness of the shadow imaging. The principles of the so-called shadow imaging are explained in Zheng et al.: "The ePetri dish, an on-chip cell imaging platform based on sub-pixel perspective sweeping microscopy (SPSM)", Proceedings of the national Academy of Sciences of the United States of America (PNAS) 2011.

Further, the thin bottom of the receptacle also allows gas diffusion through the bottom of the receptacle, so that it is not necessary to provide a conventional septum for CO2-exchange (carbonate buffer).

Moreover, the receptacle according to the invention is functionalized in order to improve the optical contrast of the imaging. In one embodiment, an upper polarization filter is arranged above the sample between the sample and a light source illuminating the sample within the receptacle from above. Further, a lower polarization filter is arranged below the sample between the sample and the image sensor viewing the sample from below. Moreover, an optical waveguide structure is arranged between the upper polarization filter and the lower polarization filter. The upper polarization filter and the lower polarization filter are aligned perpendicular to each other thereby restricting the light received by the image sensor from the light source to specific optical modes thereby achieving an improvement of the optical contrast in comparison to conventional imaging methods.

The upper polarization filter can be arranged in a cover of the receptacle, while the lower polarization filter can be arranged in the bottom of the receptacle. Further, the afore-mentioned waveguide structure can also be arranged in the bottom of the receptacle on the surface facing the sample.

In another embodiment of the invention, an upper color filter is arranged above the sample between the sample and a light source illuminating the sample from above, wherein the wavelength of the illumination from the light source is preferably within the passband of the upper color filter, so that the illumination from the light source passes through the upper color filter. Further, a lower color filter can be arranged below the sample between the sample and the image sensor viewing the sample from below, wherein the wavelength of the light emitted by the sample in response to the illumination by the light source is preferably within the passband of the lower color filter, so that the light emitted by the sample passes the lower color filter.

The upper color filter can be arranged in the cover of the receptacle, while the lower color filter can be arranged in the bottom of the receptacle.

In the afore-mentioned embodiment of the receptacle comprising upper and lower color filters, the upper side of the bottom of the receptacle is preferably coated with a pH-sensitive fluorescent dye emitting light in response to the illumination by the light source. Those parts of the pH-sensitive fluorescent dye not in contact with the sample emit light at an emission wavelength outside the passband of the lower color filter, while those parts of the pH-sensitive fluorescent dye in contact with the sample are pH-shifted by the sample thereby shifting the emission wavelength of the pH-sensitive fluorescent dye, wherein the shifted emission wavelength of the pH-sensitive fluorescent dye is within the passband of the lower color filter. In other words, the light source illuminates the sample through the upper color filter with the excitation wavelength of the pH-sensitive fluorescent dye and the image sensor detects fluorescence in those parts which are covered by the sample. In this connection, it should be noted that the passband of the upper color filter is matched to the excitation wavelength of the pH-sensitive fluorescent dye, while the passband of the lower color filter is matched to the shifted emission wavelength of the pH-sensitive fluorescent dye.

In another embodiment of the invention, the receptacle comprises at least one calibration element for optical calibration of the receptacle. The calibration element can be used for determining the transfer function of the optical system allowing a more accurate analysis of the sample within the receptacle.

In a preferred embodiment of the invention, a light source is integrated in the receptacle for illuminating the sample within the receptacle from above. For example, the light source can be arranged at least partially in the cover of the receptacle. Further, it should be noted that the light source is preferably point-shaped thereby improving the optical contrast and sharpness of the images of the sample.

In one embodiment of the invention, the light source comprises a lamp, for example a light emitting diode (LED) or an organic light emitting diode (OLED), which is preferably arranged in the cover of the receptacle. In another embodiment of the invention, the light source comprises a hole in the cover of the receptacle, wherein the sample is illuminated from above through the hole in the cover of the receptacle.

Alternatively, the light source comprises a reflecting element above the sample, particularly at the lower side of the cover, and a lamp for illuminating the reflecting element from below, wherein the reflecting element is preferably shaped as a circle or as a half-sphere.

Further, it should be noted that the invention also claims protection for a system for optically analyzing a sample, wherein the system according to the invention comprises an image sensor with a photosensitive area with a plurality of photosensitive pixels, particularly a CCD sensor or a CMOS sensor.

Moreover, the system according to the invention comprises a receptacle for receiving the sample during analysis of the sample, wherein the receptacle is preferably designed as illustrated above. In contrast to the initially mentioned conventional time-lapse microscopy, the receptacle is arranged directly on the photosensitive area of the image sensor without an optical lens between the receptacle and the image sensor. Therefore, the system according to the invention preferably allows the so-called shadow imaging without complex optics.

It should be noted that air gaps between the lower surface of the bottom of the receptacle and the photosensitive area of the image sensor cause multiple reflections and deteriorate the quality of the imaging process. These air gaps can be avoided by pressing the receptacle onto the photosensitive area. Therefore, the system according to the invention preferably comprises a pressing mechanism for pressing the receptacle onto the photosensitive area of the image sensor thereby avoiding air gaps between the receptacle and the image sensor.

Further, air gaps between the bottom of the receptacle and the photosensitive area of the image sensor can also be avoided by at least partially filling these gaps with a liquid, preferably an immersion oil or a polymer film.

Moreover, the optical resolution can be improved by moving the image sensor relative to the receptacle perpendicular to the optical axis, i.e. in the plane of the photosensitive area of the image sensors. Then, several images can be taken in different positions of the receptacle relative to the image sensor. These single images can then be used for generating an image with an improved optical resolution. Therefore, the system according to the invention preferably comprises an actuator, e.g. a piezo actuator for moving the image sensor relative to the receptacle in the plane of the photosensitive area of the image sensor in order to increase the optical resolution of the measurement. In this connection, it should be noted that the amplitude of the relative movement between the receptacle and the image sensor is preferably smaller than the distance between adjacent pixels of the image sensor.

Further, the optical resolution can also be improved by varying the direction of illumination of the sample within the receptacle. For example, the direction of illumination can be varied by providing an array of lamps or mirrors, wherein the lamps or mirrors are located at different places above the sample for successively illuminating the sample from different angles.

The optical system according to the invention preferably allows an analysis of a quite large area of a sample. Therefore, the photosensitive area of the image sensor is preferably larger than 200 mm² or 1 cm².

Further, it should be noted that the light flux passing through the sample is much smaller than the light flux passing through the sample in a light microscope so that the light exposure of the sample is reduced. This can be important for a long-term analysis of living cells which can be damaged by an intensive long-term illumination.

The concept of the invention allows small dimensions of the entire system so that the entire system can be integrated into existing working environments in laboratories. For example, the system can be integrated in a conventional incubator.

Finally, the invention preferably comprises an evaluation unit for an image-based evaluation of the images recorded by the image sensor. The evaluation unit preferably performs at least one of the following steps:
- Detection of biological cells in the images recorded by the image sensor.
- Tracking of the position of the biological cells within the mage.
- Detecting apoptosis of the biological cells.
- Detecting necrosis of the biological cells.
- Detecting mitosis of the biological cells.
- Determination of the image entropy of the image recorded by the image sensor.
- Determination of cytometric data, e.g. total number of the biological cells within the image, cell proliferation of the cells within the image, frequency of mitosis among the biological cells within the image, morphological parameters of the biological cells, particularly size, length and/or brightness of the biological cells or duration of a cell cycle.

The details of the evaluation unit are also explained in Rapoport et al.: "A novel validation algorithm allows for automated cell tracking and the extraction of biologically meaningful parameters", PLoS ONE 6(11): e27315.doi:10.1371/- journal.pone.0027315.

The invention and its particular features and advantages will become more apparent from the following detailed description considered with reference to the accompanying drawings.

### Brief description of the drawings

Figure 1 is a scheme illustrating the system according to the invention for analysis of a biological sample.
Figure 2 is a cross section of a receptacle according to the invention comprising a point-shaped light source for illumination of the sample from above.
Figure 3 is a modification of the receptacle according to figure 2 comprising a mirror in the cover for illuminating the sample.
Figure 4 shows a cross section through another modification of a receptacle comprising polarization filters both in the cover and in the bottom of the receptacle.
Figure 5 is a cross section through another modification of the embodiment according to figure 3 comprising a calibration element for optical calibration of the receptacle.
Figure 6 shows a cross section through another modification of a receptacle comprising a hole in the cover for illuminating the sample through the hole.
Figure 7A shows an air gap between the bottom of the receptacle and the image sensor.
Figure 7B shows the gap filled with a liquid in order to avoid multiple reflections.
Figure 8 shows a flowchart illustrating the operating procedure of the system according to the invention.

### Detailed description of the drawings

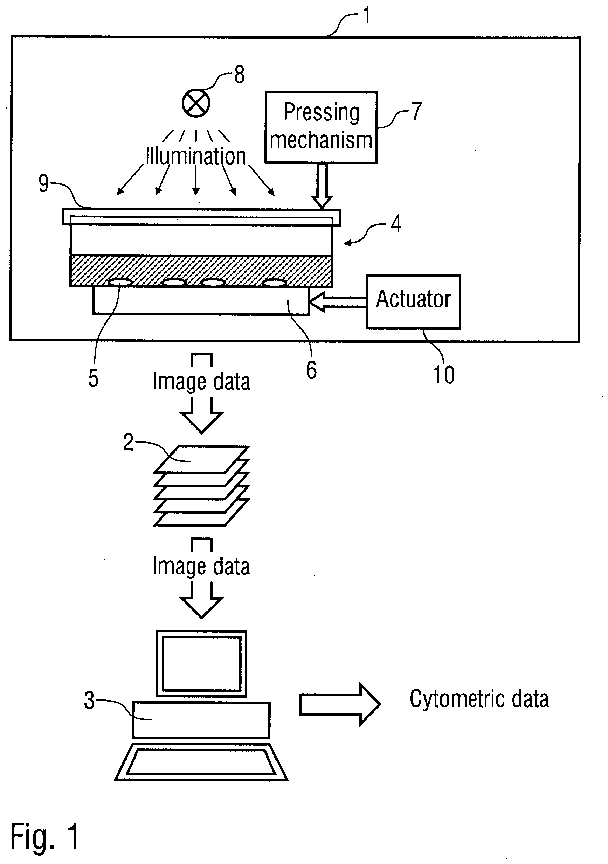

Figure 1 illustrates a system according to the invention for optically analyzing a biological sample, for example a cell culture. The system comprises an image acquisition system 1 for generating optical images 2 of the sample and an evaluation unit 3 for analyzing the images 2 and generating cytometric data.

The image acquisition system 1 comprises a receptacle 4 which includes an optical sample 5 to be analyzed, wherein the receptacle 4 comprises a transparent bottom so that the sample 5 within the receptacle 4 can be optically analyzed by an image sensor 6 from below the receptacle 4 by "shadow imaging" as explained in Zheng et al.: "The ePetri dish, an on-chip cell imaging platform based on subpixel perspective sweeping microscopy (SPSM)", Proceedings of the national Academy of Sciences of the United States of America (PNAS) 2011.

Further, it should be noted that the receptacle 4 comprises a transparent thin bottom with a thickness of less than 150 µm.

Firstly, this allows the co-called shadow imaging wherein the receptacle 4 is directly placed on the photosensitive area of the image sensor 6 without any lenses between the receptacle 4 and the image sensor 6. The thin bottom of the receptacle 4 results in an improved contrast and sharpness of the images 2 generated by the image sensor 6.

Further, the thin bottom of the receptacle 4 allows gas diffusion through the bottom, so that it is not necessary to provide a conventional septum for CO2-exchange.

Moreover, the image acquisition system 1 comprises a pressing mechanism 7 which presses the receptacle 4 onto the photosensitive area of the image sensor 6 thereby minimizing any air gaps between the lower surface of the bottom of the receptacle 4 and the photosensitive area of the image sensor 6. This is important since any air gaps between the receptacle 4 and the image sensor 6 cause multiple reflections thereby impairing the quality of the images 2.

Further, the image acquisition system 1 comprises a point-shaped light source 8 which is arranged above a removable cover 9 of the receptacle 4, so that the light source 8 illuminates the sample 5 within the receptacle 4 from above.

Moreover, the image acquisition system 1 comprises an actuator 10 (e.g. a piezo actuator) which moves the image sensor 6 relative to the receptacle 4 in the plane of the photosensitive area of the image sensor 6, i.e. perpendicular to the optical axis. Then, the image sensor 6 takes several images of the sample 5 in different positions of the image sensor 6 relative to the receptacle 4. This allows the evaluation unit 3 to calculate a resulting image with a higher optical resolution. In other words, the sub-pixel movements of the image sensor 6 relative to the receptacle 4 improve the effective optical resolution.

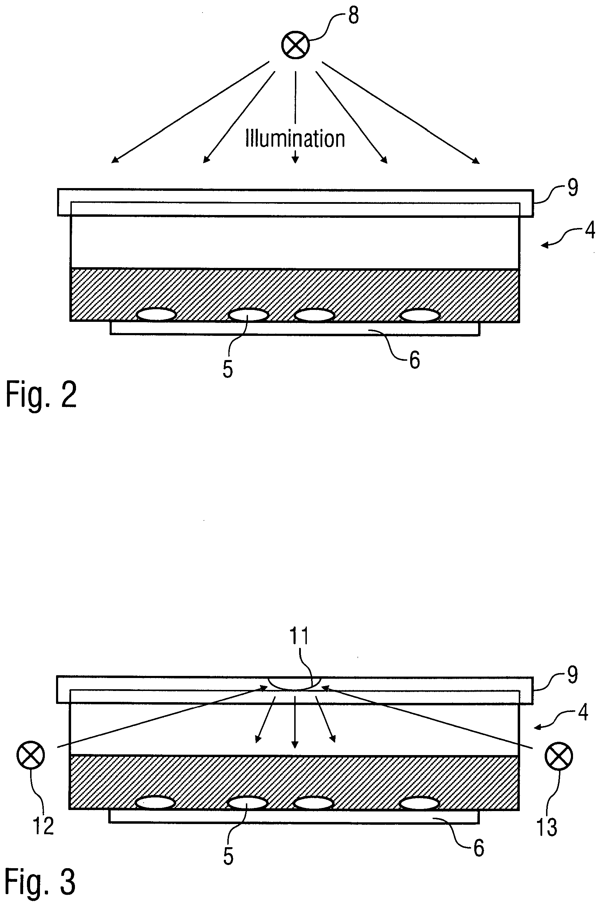

Figure 2 shows a cross section through the receptacle 4 of the image acquisition system 1 of figure 1 along with the point-shaped light source 8.

Figure 3 shows a modification of figure 2 so that reference is made to the above description, wherein the same reference signs are used to designate corresponding details.

Instead of the point-shaped light source 8, the embodiment of figure 3 comprises a reflecting element 11 (i.e. a mirror) which is arranged on the lower side of the cover 9 of the receptacle 4, wherein the reflecting element 11 is illuminated by two light sources 12, 13 which are arranged on opposite sides of the receptacle 4. Therefore, the sample 5 within the receptacle 4 can be illuminated from different directions either by the light source 12 or by the light source 13. The image acquisition system 1 takes images of the sample 5 with different directions of illuminations, which allows the evaluation unit 3 to calculate a resulting image with an improved optical resolution.

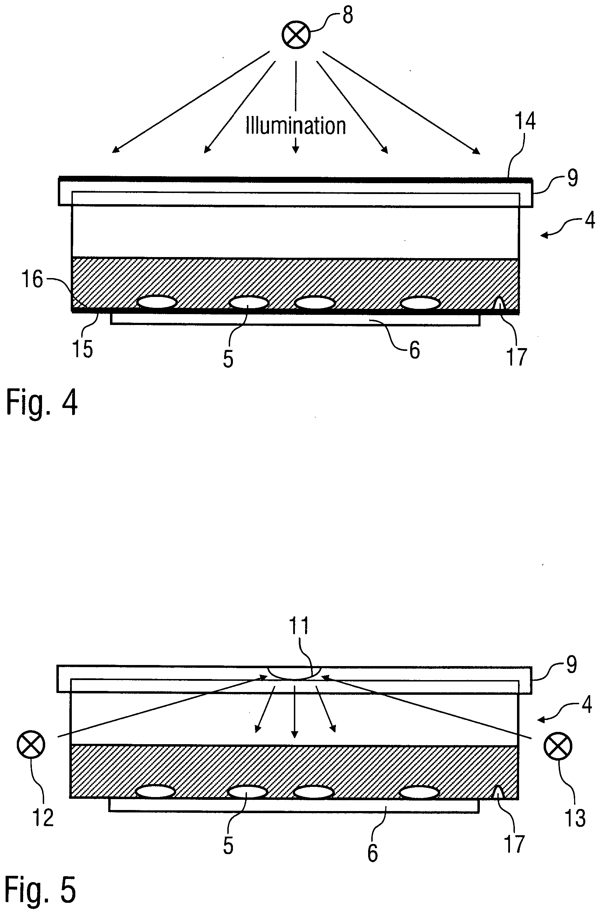

Figure 4 shows another modification of the receptacle 4 as shown in figure 2, so that reference is made to the above description, wherein the same reference signs are used to designate corresponding details.

In this embodiment of the invention, an upper polarization filter 14 is arranged in the cover 9 of the receptacle 4. Further, a lower polarization filter 15 is arranged in the bottom of the receptacle 4, wherein the upper polarization filter 14 and the lower polarization filter 15 are aligned perpendicular to each other. Further, an optical waveguide structure 16 is applied to the upper surface of the bottom of the receptacle 4. The combination of the lower and upper polarization filters 14, 15 and the optical waveguide structure 16 improves the optical contrast as explained in Nazirizadeh, Y.: "Photonic crystal slabs for surface contrast enhancement in microscopy of transparent objects", Optics Express, Vol. 20, Issue 13, pp. 14451-14459 (2012).

Further, the receptacle 4 of figure 4 comprises a calibration element 17 being arranged on the upper side of the bottom of the receptacle 4. The calibration element 17 allows a measurement of the transfer function of the data acquisition system 1 which in turn allows an improvement of the optical resolution.

Figure 5 largely corresponds to figure 3 and additionally comprises the calibration element 17 as mentioned above.

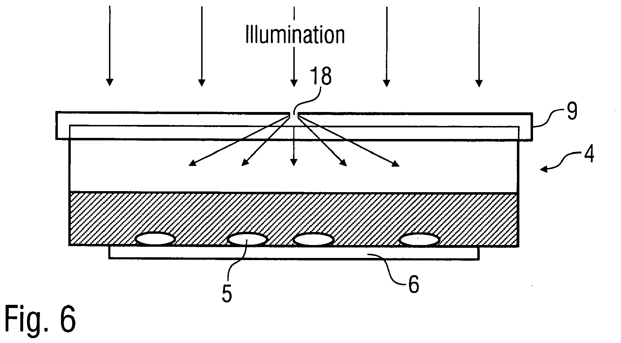

Figure 6 shows a further modification of the receptacle 4 as shown in figure 2-5. Instead of the light source 8, the receptacle 4 comprises a hole 18 in the center of the cover 9, so that the sample 5 in the receptacle 4 is illuminated by ambient light through the hole 18.

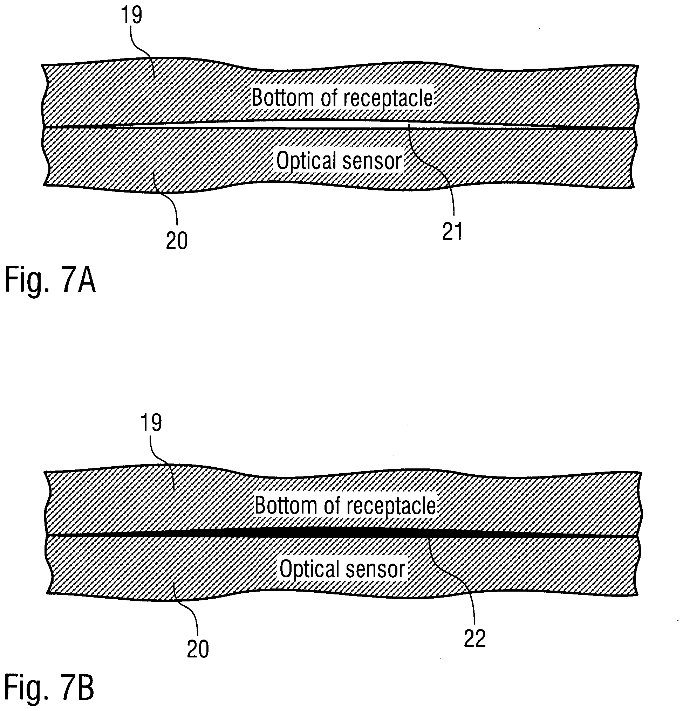

Figure 7A shows a cross section through a bottom 19 of the receptacle 4 being arranged on a photosensitive surface 20 of the image sensor 6. The cross section shows that there is an air gap 21 between the bottom 19 of the receptacle 4 and the photosensitive surface 20 of the image sensor 6. However, the air gap 21 causes multiple reflections thereby impairing the quality of the images 2.

Figure 7B shows an improvement of figure 7A, wherein the air gap 21 is filled with an immersion oil 22, so that multiple reflections are avoided thereby improving the quality of the images.

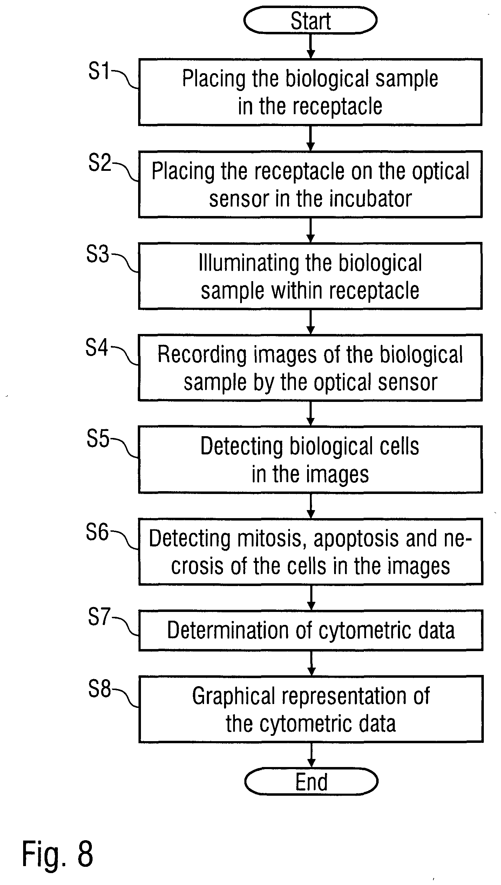

Figure 8 shows a flow chart illustrating the operating method of the system as shown in figure 1.

In a first step S1, the biological sample 5 is placed in the receptacle 4 on the bottom of the receptacle 4.

In a next step S2, the receptacle 4 is placed on the photosensitive surface 20 of the image sensor 6 in the incubator, wherein the incubator is not shown in the drawings.

Then, the biological sample 5 in the receptacle 4 is illuminated in step S3 and the images 2 of the biological sample 5 are recorded by the image sensor 6 in step S4.

The evaluation unit 3 then detects biological cells in the images 2 in step S5.

In a following step S6 the evaluation unit 3 detects mitosis, apoptosis and necrosis of the cells in the images 2.

In another step S7, the evaluation unit 3 determines cytometric data relating to the cells shown in the images 2.

Finally, the cytometric data are graphically represented in step S8.

Although the invention has been described with reference to the particular arrangement of parts, features and the like, these are not intended to exhaust all possible arrangements of features and indeed many other modifications and variations will be ascertainable to those of skill in the art.

### List of reference signs:

- 1: Image acquisition system
- 2: Images
- 3: Evaluation unit
- 4: Receptacle
- 5: Sample
- 6: Image sensor
- 7: Pressing mechanism
- 8: Light source
- 9: Cover
- 10: Actuator
- 11: Reflecting element
- 12: Light source
- 13: Light source
- 14: Upper polarization
- 15: Lower polarization filter
- 16: Optical waveguide structure
- 17: Calibration element
- 18: Hole in the cover
- 19: Bottom of the receptacle
- 20: Photosensitive surface of the optical sensor
- 21: Air gap
- 22: Immersion oil

## Claims

1. Receptacle (4) for receiving a sample (5) during an optical analysis of the sample (5),
**characterized in that**
a) the receptacle (4) comprises a bottom (19) which is at least partially transparent so that the sample (5) within the receptacle (4) can be optically analysed by an image sensor (6) from below the bottom (19),
b) the bottom (19) of the receptacle (4) comprises a thickness of less than 500µm, 200µm, 150µm or even less than 120µm,
c) the thickness of the bottom (19) of the receptacle (4) is sufficiently small to allow gas diffusion through the bottom (19),
d) an upper filter (14) is arranged above the sample (5) between the sample (5) and a light source illuminating the sample (5) from above, and a lower filter (15) is arranged below the sample (5) between the sample (5) and the image sensor (6) viewing the sample (5) from below,
e) wherein
e1) the upper filter (14) is an upper polarization filter (14) and the lower filter (15) is a lower polarization filter (15), and an optical wave guide structure (16) is arranged between the upper polarization filter (14) and the lower polarization filter (15), and the upper polarization filter (14) and the lower polarization filter (15) are aligned perpendicular to each other thereby restricting the light received by the image sensor (6) from the light source to specific optical modes, or
e2) the upper filter (14) is an upper color filter (14) and the lower filter (15) is a lower color filter (15) .

2. Receptacle (4) according to claim 1, wherein
a) the wavelength of the illumination from the light source (8) is within the passband of the upper color filter, so that the illumination from the light source (8) passes the upper color filter, and
b) the wavelength of light emitted by the sample (5) in response to the illumination by the light source (8) is within the passband of the lower color filter, so that the light emitted by the sample (5) passes the lower color filter.

3. Receptacle (4) according to claim 1 or claim 2,
wherein
a) the upper side of the bottom (19) of the receptacle (4) is coated with a pH-sensitive fluorescent dye emitting light in response to the illumination by the light source, and
b) those parts of the pH-sensitive fluorescent dye not in contact with the sample (5) emit light at an emission wavelength outside the passband of the lower color filter,
c) those parts of the pH-sensitive fluorescent dye in contact with the sample (5) are pH-shifted by the sample (5) thereby shifting the emission wavelength of the pH-sensitive fluorescent dye, wherein the shifted emission wavelength of the pH-sensitive fluorescent dye is within the passband of the lower color filter.

4. Receptacle (4) according to any of claims 1 to 3, wherein
a) the upper polarisation filter (14) is arranged in a cover (9) of the receptacle (4), and/or
b) the lower polarisation filter (15) is arranged in the bottom (19) of the receptacle (4), and/or
c) the upper color filter is arranged in a cover (9) of the receptacle (4), and/or
d) the lower color filter is arranged in the bottom (19) of the receptacle (4).

5. Receptacle (4) according to any of the preceding claims, further comprising a calibration element (17) for optical calibration.

6. Receptacle (4) according to any of the preceding claims, wherein
a) a light source (8, 11, 12, 13, 18) is integrated in the receptacle (4) for illuminating the sample (5) within the receptacle (4) from above, and/or
b) the receptacle (4) comprises a cover (9), wherein the light source (8, 11, 12, 13, 18) is arranged at least partially in the cover (9), and/or
c) the light source (8, 11, 18) is point-shaped.

7. Receptacle (4) according to claim 6, wherein
a) the light source (8, 11, 12, 13,18) comprises a lamp (8, 12, 13) arranged in the cover (9) of the receptacle (4), or
b) the light source (8, 11, 12, 13, 18) comprises a hole (18) in the cover (9) of the receptacle (4), or
c) the light source (8, 11, 12, 13, 18) comprises a reflecting element (11) above the sample (5) and a lamp (12, 13) for illuminating the reflecting element (11) from below, wherein the reflecting element (11) is shaped as a circle or as a half-sphere.

8. System for optically analysing a sample (5), comprising:
a) an image sensor (6) comprising a photosensitive area (20) with a plurality of photosensitive pixels, and
b) a receptacle (4) according to any of the preceding claims for receiving the sample (5) during analysis of the sample (5),
c) wherein the receptacle (4) is arranged directly on the photosensitive area (20) of the image sensor (6) without any optical lens between the receptacle (4) and the image sensor (6).

9. System according to claim 8, further comprising a pressing mechanism (7) for pressing the receptacle (4) onto the photosensitive area (20) of the image sensor (6) for avoiding an air gap (21) between the photosensitive area (20) of the image sensor (6) on the one hand and the bottom (19) of the receptacle (4) on the other hand.

10. System according to any of claims 8 to 9, wherein any gap (21) between the bottom (19) of the receptacle (4) and the photosensitive area (20) of the image sensor (6) is at least partially filled with a liquid (22).

11. System according to any of claims 8 to 10, further comprising an actuator (10) for moving the image sensor (6) relative to the receptacle (4) in the plane of the photosensitive area (20) of the image sensor (6) in order to increase the optical resolution of the measurement,
wherein the amplitude of the relative movement is smaller than the distance between adjacent pixels of the image sensor (6) .

12. System according to any of claims 8 to 11, further comprising means (11, 12, 13) for varying a direction of illumination of the sample (5) within the receptacle (4).

13. System according to claim 12, wherein the means (11, 12, 13) for varying the direction of illumination comprises an array of lamps (12, 13) and/or mirrors (11), wherein the lamps (12, 13) or mirrors (11) are located at different places above the sample (5) for successively illuminating the sample (5) from different angles.

## Patentansprüche

1. Behälter (4) zur Aufnahme einer Probe (5) während einer optischen Analyse der Probe (5),
**dadurch gekennzeichnet, dass**
a) der Behälter (4) einen Boden (19) aufweist, der zumindest teilweise transparent ist, so dass die Probe (5) innerhalb des Behälters (4) durch einen Bildsensor (6) von unterhalb des Bodens (19) optisch analysiert werden kann,
b) der Boden (19) des Behälters (4) eine Dicke von weniger als 500 pm, 200 pm, 150 µm oder sogar weniger als 120 µm aufweist,
c) die Dicke des Bodens (19) des Behälters (4) ausreichend gering ist, um eine Gasdiffusion durch den Boden (19) zu ermöglichen,
d) ein oberer Filter (14) oberhalb der Probe (5) zwischen der Probe (5) und einer Lichtquelle, die die Probe (5) von oben beleuchtet, angeordnet ist und ein unterer Filter (15) unterhalb der Probe (5) zwischen der Probe (5) und dem Bildsensor (6), der die Probe (5) von unten betrachtet, angeordnet ist,
e) wobei
e1) der obere Filter (14) ein oberer Polarisationsfilter (14) und der untere Filter (15) ein unterer Polarisationsfilter (15) ist, und eine optische Wellenleiterstruktur (16) zwischen dem oberen Polarisationsfilter (14) und dem unteren Polarisationsfilter (15) angeordnet ist, und der obere Polarisationsfilter (14) und der untere Polarisationsfilter (15) senkrecht zueinander ausgerichtet sind, wodurch das von dem Bildsensor (6) von der Lichtquelle empfangene Licht auf bestimmte optische Modi beschränkt wird, oder
e2) der obere Filter (14) ein oberer Farbfilter (14) und der untere Filter (15) ein unterer Farbfilter (15) ist.

2. Behälter (4) nach Anspruch 1, wobei
a) die Wellenlänge der Beleuchtung von der Lichtquelle (8) innerhalb des Durchlassbereichs des oberen Farbfilters liegt, so dass die Beleuchtung von der Lichtquelle (8) den oberen Farbfilter passiert, und
b) die Wellenlänge des von der Probe (5) als Reaktion auf die Beleuchtung durch die Lichtquelle (8) emittierten Lichts innerhalb des Durchlassbereichs des unteren Farbfilters liegt, so dass das von der Probe (5) emittierte Licht den unteren Farbfilter passiert.

3. Behälter (4) nach Anspruch 1 oder Anspruch 2,
wobei
a) die Oberseite des Bodens (19) des Behälters (4) mit einem pH-empfindlichen Fluoreszenzfarbstoff beschichtet ist, der als Reaktion auf die Beleuchtung durch die Lichtquelle Licht emittiert, und
b) die Teile des pH-empfindlichen Fluoreszenzfarbstoffs, die nicht in Kontakt mit der Probe (5) sind, Licht bei einer Emissionswellenlänge außerhalb des Durchlassbereichs des unteren Farbfilters emittieren,
c) die Teile des pH-empfindlichen Fluoreszenzfarbstoffs, die mit der Probe (5) in Kontakt sind, durch die Probe (5) pH-verschoben werden, wodurch die Emissionswellenlänge des pH-empfindlichen Fluoreszenzfarbstoffs verschoben wird, wobei die verschobene Emissionswellenlänge des pH-empfindlichen Fluoreszenzfarbstoffs innerhalb des Durchlassbereichs des unteren Farbfilters liegt.

4. Behälter (4) nach einem der Ansprüche 1 bis 3, wobei
a) der obere Polarisationsfilter (14) in einem Deckel (9) des Behälters (4) angeordnet ist, und/oder
b) der untere Polarisationsfilter (15) im Boden (19) des Behälters (4) angeordnet ist, und/oder
c) der obere Farbfilter in einem Deckel (9) des Behälters (4) angeordnet ist, und/oder
d) der untere Farbfilter im Boden (19) des Behälters (4) angeordnet ist.

5. Behälter (4) nach einem der vorhergehenden Ansprüche, ferner umfassend ein Kalibrierelement (17) zur optischen Kalibrierung.

6. Behälter (4) nach einem der vorhergehenden Ansprüche, wobei
a) eine Lichtquelle (8, 11, 12, 13, 18) in den Behälter (4) integriert ist, um die Probe (5) innerhalb des Behälters (4) von oben zu beleuchten, und/oder
b) der Behälter (4) einen Deckel (9) aufweist, wobei die Lichtquelle (8, 11, 12, 13, 18) zumindest teilweise in dem Deckel (9) angeordnet ist, und/oder
c) die Lichtquelle (8, 11, 18) punktförmig ist.

7. Behälter (4) nach Anspruch 6, wobei
a) die Lichtquelle (8, 11, 12, 13, 18) eine in dem Deckel (9) des Behälters (4) angeordnete Lampe (8, 12, 13) umfasst, oder
b) die Lichtquelle (8, 11, 12, 13, 18) ein Loch (18) in dem Deckel (9) des Behälters (4) aufweist, oder
c) die Lichtquelle (8, 11, 12, 13, 18) ein reflektierendes Element (11) oberhalb der Probe (5) und eine Lampe (12, 13) zur Beleuchtung des reflektierenden Elements (11) von unten aufweist, wobei das reflektierende Element (11) als Kreis oder als Halbkugel ausgebildet ist.

8. System zur optischen Analyse einer Probe (5), das Folgendes umfasst:
a) einen Bildsensor (6), der eine lichtempfindliche Fläche (20) mit einer Vielzahl von lichtempfindlichen Pixeln aufweist, und
b) einen Behälter (4) nach einem der vorhergehenden Ansprüche zur Aufnahme der Probe (5) während der Analyse der Probe (5),
c) wobei der Behälter (4) direkt auf der lichtempfindlichen Fläche (20) des Bildsensors (6) angeordnet ist, ohne dass sich zwischen dem Behälter (4) und dem Bildsensor (6) eine optische Linse befindet.

9. System nach Anspruch 8, ferner umfassend einen Andruckmechanismus (7) zum Andrücken des Behälters (4) auf die lichtempfindliche Fläche (20) des Bildsensors (6), um einen Luftspalt (21) zwischen der lichtempfindlichen Fläche (20) des Bildsensors (6) einerseits und dem Boden (19) des Behälters (4) andererseits zu vermeiden.

10. System nach einem der Ansprüche 8 bis 9, wobei ein etwaiger Spalt (21) zwischen dem Boden (19) des Behälters (4) und dem lichtempfindlichen Bereich (20) des Bildsensors (6) zumindest teilweise mit einer Flüssigkeit (22) gefüllt ist.

11. System nach einem der Ansprüche 8 bis 10, ferner mit einem Aktuator (10) zum Bewegen des Bildsensors (6) relativ zum Behälter (4) in der Ebene der lichtempfindlichen Fläche (20) des Bildsensors (6), um die optische Auflösung der Messung zu erhöhen,
wobei die Amplitude der Relativbewegung kleiner ist als der Abstand zwischen benachbarten Pixeln des Bildsensors (6).

12. System nach einem der Ansprüche 8 bis 11, ferner mit Mitteln (11, 12, 13) zum Variieren einer Beleuchtungsrichtung der Probe (5) innerhalb des Behälters (4).

13. System nach Anspruch 12, wobei die Mittel (11, 12, 13) zum Variieren der Beleuchtungsrichtung eine Anordnung von Lampen (12, 13) und/oder Spiegeln (11) umfassen, wobei die Lampen (12, 13) oder Spiegel (11) an verschiedenen Stellen über der Probe (5) angeordnet sind, um die Probe (5) nacheinander aus verschiedenen Winkeln zu beleuchten.

## Revendications

1. Réceptacle (4) pour recevoir un échantillon (5) au cours d'une analyse optique de l'échantillon (5),
**caractérisé en ce que**
a) le réceptacle (4) comprend un fond (19) qui est au moins partiellement transparent de sorte que l'échantillon (5) à l'intérieur du réceptacle (4) puisse être analysé optiquement par un capteur d'image (6) par-dessous le fond (19),
b) le fond (19) du réceptacle (4) comprend une épaisseur inférieure à 500 pm, 200 pm, 150 pm, ou même inférieure à 120 pm,
c) l'épaisseur du fond (19) du réceptacle (4) est suffisamment petite pour permettre une diffusion de gaz à travers le fond (19),
d) un filtre supérieur (14) est agencé au-dessus de l'échantillon (5) entre l'échantillon (5) et une source lumineuse éclairant l'échantillon (5) par-dessus, et un filtre inférieur (15) est agencé au-dessous de l'échantillon (5) entre l'échantillon (5) et le capteur d'image (6) visualisant l'échantillon (5) par-dessous,
e) dans lequel
e1) le filtre supérieur (14) est un filtre supérieur de polarisation (14) et le filtre inférieur (15) est un filtre inférieur de polarisation (15), et une structure de guide d'onde optique (16) est agencée entre le filtre supérieur de polarisation (14) et le filtre inférieur de polarisation (15), et le filtre supérieur de polarisation (14) et le filtre inférieur de polarisation (15) sont alignés perpendiculairement l'un par rapport à l'autre en limitant de ce fait la lumière reçue par le capteur d'image (6) depuis la source lumineuse à des modes optiques spécifiques, ou
e2) le filtre supérieur (14) est un filtre supérieur de couleur (14) et le filtre inférieur (15) est un filtre inférieur de couleur (15).

2. Réceptacle (4) selon la revendication 1, dans lequel
a) la longueur d'onde de l'éclairage depuis la source lumineuse (8) est à l'intérieur de la bande passante du filtre supérieur de couleur, de sorte que l'éclairage depuis la source lumineuse (8) passe par le filtre supérieur de couleur, et
b) la longueur d'onde de la lumière émise par l'échantillon (5) en réponse à l'éclairage par la source lumineuse (8) est à l'intérieur de la bande passante du filtre inférieur de couleur, de sorte que la lumière émise par l'échantillon (5) passe par le filtre inférieur de couleur.

3. Réceptacle (4) selon la revendication 1 ou 2, dans lequel
a) le côté supérieur du fond (19) du réceptacle (4) est revêtu d'un colorant fluorescent sensible au pH émettant une lumière en réponse à l'éclairage par la source lumineuse, et
b) les parties du colorant fluorescent sensible au pH qui ne sont pas en contact avec l'échantillon (5) émettent une lumière à une longueur d'onde d'émission à l'extérieur de la bande passante du filtre inférieur de couleur,
c) les parties du colorant fluorescent sensible au pH qui sont en contact avec l'échantillon (5) subissent un décalage de pH par l'échantillon (5), ce qui décale la longueur d'onde d'émission du colorant fluorescent sensible au pH, dans lequel la longueur d'onde d'émission décalée du colorant fluorescent sensible au pH est à l'intérieur de la bande passante du filtre inférieur de couleur.

4. Réceptacle (4) selon l'une quelconque des revendications 1 à 3, dans lequel
a) le filtre supérieur de polarisation (14) est agencé dans un couvercle (9) du réceptacle (4), et/ou
b) le filtre inférieur de polarisation (15) est agencé dans le fond (19) du réceptacle (4), et/ou
c) le filtre supérieur de couleur est agencé dans un couvercle (9) du réceptacle (4), et/ou
d) le filtre inférieur de couleur est agencé dans le fond (19) du réceptacle (4).

5. Réceptacle (4) selon l'une quelconque des revendications précédentes, comprenant en outre un élément d'étalonnage (17) pour un étalonnage optique.

6. Réceptacle (4) selon l'une quelconque des revendications précédentes, dans lequel
a) une source lumineuse (8, 11, 12, 13, 18) est intégrée dans le réceptacle (4) pour éclairer l'échantillon (5) à l'intérieur du réceptacle (4) par-dessus, et/ou
b) le réceptacle (4) comprend un couvercle (9), dans lequel la source lumineuse (8, 11, 12, 13, 18) est agencée au moins partiellement dans le couvercle (9), et/ou
c) la source lumineuse (8, 11, 18) est ponctuelle.

7. Réceptacle (4) selon la revendication 6, dans lequel
a) la source lumineuse (8, 11, 12, 13, 18) comprend une lampe (8, 12, 13) agencée dans le couvercle (9) du réceptacle (4), ou
b) la source lumineuse (8, 11, 12, 13, 18) comprend un trou (18) dans le couvercle (9) du réceptacle (4), ou
c) la source lumineuse (8, 11, 12, 13, 18) comprend un élément réfléchissant (11) au-dessus de l'échantillon (5) et une lampe (12, 13) pour éclairer l'élément réfléchissant (11) par-dessous, dans lequel l'élément réfléchissant (11) est en forme de cercle ou de demi- sphère.

8. Système pour analyser optiquement un échantillon (5), comprenant :
a) un capteur d'image (6) comprenant une zone photosensible (20) avec une pluralité de pixels photosensibles, et
b) un réceptacle (4) selon l'une quelconque des revendications précédentes pour recevoir l'échantillon (5) au cours d'une analyse de l'échantillon (5),
c) dans lequel le réceptacle (4) est agencé directement sur la zone photosensible (20) du capteur d'image (6) sans aucune lentille optique entre le réceptacle (4) et le capteur d'image (6).

9. Système selon la revendication 8, comprenant en outre un mécanisme de pression (7) pour presser le réceptacle (4) sur la zone photosensible (20) du capteur d'image (6) pour éviter tout écartement d'air (21) entre la zone photosensible (20) du capteur d'image (6) d'une part et le fond (19) du réceptacle (4) d'autre part.

10. Système selon la revendication 8 ou 9, dans lequel tout écartement (21) entre le fond (19) du réceptacle (4) et la zone photosensible (20) du capteur d'image (6) est au moins partiellement rempli d'un liquide (22).

11. Système selon l'une quelconque des revendications 8 à 10, comprenant en outre un actionneur (10) pour déplacer le capteur d'image (6) par rapport au réceptacle (4) dans le plan de la zone photosensible (20) du capteur d'image (6) afin d'augmenter la résolution optique de la mesure,
dans lequel l'amplitude du déplacement relatif est plus petite que la distance entre des pixels adjacents du capteur d'image (6).

12. Système selon l'une quelconque des revendications 8 à 11, comprenant en outre des moyens (11, 12, 13) pour changer une direction d'éclairage de l'échantillon (5) à l'intérieur du réceptacle (4).

13. Système selon la revendication 12, dans lequel les moyens (11, 12, 13) pour changer la direction d'éclairage comprennent un réseau de lampes (12, 13) et/ou de miroirs (11), dans lequel les lampes (12, 13) ou les miroirs (11) sont situés à des emplacements différents au-dessus de l'échantillon (5) pour éclairer successivement l'échantillon (5) à des angles différents.
